# EUROPÄISCHE PATENTSCHRIFT

(11) **EP 4 273 537 B1**
(45) Veröffentlichungstag und Bekanntmachung des Hinweises auf die Patenterteilung: **12.03.2025**
(21) Anmeldenummer: 23169133.8
(22) Anmeldetag: 21.04.2023
(51) Int. Cl.: G01N 27/404, G01N 33/00

(54) **ELEKTROCHEMISCHER SENSOR MIT EINER MESSZELLE UND EINEM OXIDIERUNGS-BAUTEIL UND VERFAHREN UNTER VERWENDUNG EINES SOLCHEN SENSORS**
ELECTROCHEMICAL SENSOR COMPRISING A MEASURING CELL AND AN OXIDATION COMPONENT AND METHOD USING SUCH A SENSOR
CAPTEUR ÉLECTROCHIMIQUE COMPRENANT UNE CELLULE DE MESURE ET UN COMPOSANT OXYDANT ET PROCÉDÉ UTILISANT UN TEL CAPTEUR

(30) Priorität: 06.05.2022 DE 102022111351
(43) Veröffentlichungstag der Anmeldung: 08.11.2023
(73) Patentinhaber: Dräger Safety AG & Co. KGaA, 23560 Lübeck (DE)
(72) Erfinder: Sick, Michael, 23558 Lübeck (DE); Wolter, Christopher, 23558 Lübeck (DE); Eckhardt, Rolf, 23558 Lübeck (DE)
(74) Vertreter: Meyer-Gramann, Klaus Dieter

(56) Entgegenhaltungen:
- EP-A2- 0 301 897
- JP-A- 2005 083 956
- JP-A- 2016 164 507
- US-A1- 2003 029 721

## Beschreibung

Die Erfindung betrifft einen elektrochemischen Sensor mit einer Messzelle, wobei die Messzelle einen vorgegebenen Gas-Bestandteil in einem Gas zu detektieren und / oder die Konzentration des vorgegebenen Gas-Bestandteils, beispielsweise Sauerstoff, in dem Gas zu messen vermag. Weiterhin betrifft die Erfindung ein Verfahren, bei welchem ein derartiger Sensor verwendet wird.

Ein elektrochemischer Sauerstoffsensor 1 wird in EP 2002251 B1 beschrieben. Ein Gehäuse 12 umschließt eine Kammer, in der eine Messzelle mit einer Messelektrode 6, einer Gegenelektrode 5, einer Referenzelektrode 9 und einem Elektrolyten angeordnet ist. Zwischen der Messelektrode 6 und der Gegenelektrode 5 ist ein impermeables Isolierelement 11 angeordnet. Ein Gas, das auf Sauerstoff zu untersuchen ist, kann durch eine erste Öffnung 2 die Messelektrode 6 erreichen. Durch eine zweite Öffnung 3 kann das Gas wieder aus der Kammer entweichen. Die beiden Öffnungen 2 und 3 sind auf derselben Seite des Gehäuses 12 angeordnet.

Der elektrochemische Sensor von JP 2005083956 A umfasst einen Kanal mit einem Einlass 32, einer Kammer 31 und einem Auslass 33, durch den hindurch eine zu untersuchende Gasprobe fließen kann. Wenigstens ein Teil der Gasprobe, die durch diesen Kanal hindurchfließt, erreicht durch poröse Membranen 18, 17 und durch ein Bauteil 16 hindurch eine Messelektrode 10 einer Messzelle. Die Messzelle umfasst weiterhin eine Gegenelektrode 5 und eine Referenzelektrode 6 sowie einen Elektrolyten 20 zwischen der Messelektrode 10 auf der einen Seite und den beiden Elektroden 5 und 6 auf der anderen Seite. Das Bauteil 16 bewirkt, dass H2S in der Gasprobe durch eine chemische Reaktion oxidiert wird. Luft aus der Umgebung kann durch eine Öffnung 30 hindurch die beiden Elektroden 5 und 6 erreichen.

Der Erfindung liegt die Aufgabe zugrunde, einen elektrochemischen Sensor mit den Merkmalen des Oberbegriffs des Anspruchs 1 und ein Verfahren mit den Merkmalen des Oberbegriffs des Anspruchs 9 bereitzustellen, die weniger empfindlich gegen Umwelteinflüsse als bekannte elektrochemische Sensoren und Verfahren sind.

Die Aufgabe wird durch einen elektrochemischen Sensor mit den Merkmalen des Anspruchs 1 und durch ein Verfahren mit den Merkmalen des Anspruchs 9 gelöst. Vorteilhafte Ausgestaltungen des erfindungsgemäßen elektrochemischen Sensors sind, soweit sinnvoll, auch vorteilhafte Ausgestaltungen des erfindungsgemäßen Verfahrens und umgekehrt.

Der erfindungsgemäße elektrochemische Sensor umfasst ein Gehäuse. Dieses Gehäuse umschließt einen Innenraum. Ein messzellenseitiger Einlass vermag wenigstens zeitweise eine Fluidverbindung zwischen dem den Innenraum und einer Umgebung des elektrochemischen Sensors herzustellen. Außerdem steht der Innenraum wenigstens zeitweise über einen Druckausgleichs-Auslass mit der Umgebung in einer Fluidverbindung.

Der erfindungsgemäße elektrochemische Sensor umfasst weiterhin eine Messzelle. Die Messzelle ist zwischen dem messzellenseitigen Einlass und dem Druckausgleichs-Auslass angeordnet. Ein zu untersuchendes Gas kann durch den messzellenseitigen Einlass hindurch zur Messzelle gelangen. Dieses Gas kann mindestens einen vorgegebenen Gas-Bestandteil aufweisen. Dieser Gas-Bestandteil ist zu detektieren und wird oft auch als Zielgas bezeichnet.

Die Messzelle umfasst
- eine Messelektrode,
- eine Gegenelektrode und
- eine Schicht zwischen den beiden Elektroden.

Einerseits ist die Schicht zwischen den beiden Elektroden der Messzelle elektrisch isolierend und vermeidet insbesondere einen Kurzschluss zwischen den beiden Elektroden. Andererseits ist die Schicht ionisch leitend, ermöglicht also, dass Ionen von der einen Elektrode zu der anderen Elektrode fließen.

An oder in der Messzelle findet eine chemische Reaktion statt. Diese chemische Reaktion hängt davon ab, wie groß die Konzentration des zu detektierenden Gas-Bestandteils im Innenraum ist. Falls im zu untersuchenden Gas mehrere zu detektierende Gas-Bestandteile vorhanden sind, so hängt die chemische Reaktion in der Regel von den summierten Konzentrationen dieser Gas-Bestandteile ab.

Die chemische Reaktion beeinflusst eine messbare elektrische Größe, beispielsweise die Stromstärke oder die elektrische Ladung (aufsummierte Stromstärke) oder die elektrische Spannung. Eine elektrische Messeinheit des Sensors vermag ein Maß für diese beeinflusste oder beeinflussbare elektrische Größe zu messen. Unter einem "elektrochemischen Sensor" wird ein Sensor mit einer derartigen Messzelle verstanden.

Weiterhin umfasst der elektrochemische Sensor ein Oxidierungs-Bauteil. Dieses Oxidierungs-Bauteil umfasst mindestens eine Elektrode. Das Oxidierungs-Bauteil ist zwischen dem Druckausgleichs-Auslass und der Messzelle angeordnet. Zwischen der Messzelle und dem Oxidierungs-Bauteil tritt ein räumlicher Abstand auf. Das Oxidierungs-Bauteil ist elektrisch leitend und elektrisch von der Messzelle isoliert. Bevorzugt ist mindestens eine Komponente des Oxidierungs-Bauteils, beispielsweise die Elektrode, elektrisch leitend. Bevorzugt ist das Oxidierungs-Bauteil ionisch leitend mit der Messzelle verbunden.

Das Oxidierungs-Bauteil vermag mindestens einen, bevorzugt jeden oxidierbaren Gas-Bestandteil im Gas zu oxidieren, idealerweise vollständig zu oxidieren, wobei dieser Gas-Bestandteil als Teil des zu untersuchenden Gases durch den Druckausgleichs-Auslass hindurch in den Innenraum fließt oder geflossen ist. Weil der Gas-Bestandteil oxidiert ist, erreicht er nicht mehr die Messzelle.

Das erfindungsgemäße Verfahren wird unter Verwendung eines erfindungsgemäßen elektrochemischen Sensors durchgeführt und umfasst die folgenden Schritte:
- Durch den messzellenseitigen Einlass hindurch fließt eine Probe des zu untersuchenden Gases in den Innenraum, insbesondere durch Diffusion und / oder Ansaugen, und erreicht die Messzelle.
- An und / oder in der Messzelle findet eine chemische Reaktion statt. Diese chemische Reaktion hängt von der Konzentration des zu detektierenden Gas-Bestandteils im Innenraum ab. Die chemische Reaktion beeinflusst eine messbare elektrische Größe.
- Die elektrische Messeinheit misst ein Maß für diese elektrische Größe.
- Möglich ist, dass Gas durch den Druckausgleichs-Auslass hindurch in den Innenraum gelangt. Das Oxidierungs-Bauteil oxidiert mindestens einen, bevorzugt jeden oxidierbaren Gas-Bestandteil, wobei der oder jeder oxidierte Gas-Bestandteil zu dem Gas gehört, welches durch den Druckausgleichs-Auslass hindurch in den Innenraum gelangt.

Die Messeinheit der Messzelle misst ein Maß für die elektrische Größe, die von der chemischen Reaktion in der Messzelle beeinflusst wird. Diese chemische Reaktion wird von mindestens einem zu detektierenden Gas-Bestandteil, welches in dem zu untersuchenden Gas in der Umgebung vorhanden sein kann, kann, bewirkt und / oder aufrecht erhalten. In der Regel wird kein Bestandteil des elektrochemischen Sensors, insbesondere kein Bestandteil der elektrischen Messeinheit, im Laufe eines Einsatzes signifikant verbraucht. Daher ist es häufig nicht erforderlich, den Füllstand einer Substanz zu überwachen und bei Bedarf diese Substanz nachzufüllen.

In vielen Fällen verbraucht die Messzelle weniger elektrische Energie als eine denkbare andere Ausgestaltung eines elektrochemischen Sensors und als eine optionale signalverarbeitende Auswerteeinheit und eine optionale Anzeigeeinheit. Oft verbrauchen die Messzelle und die Messeinheit überhaupt keine elektrische Energie, solange kein zu detektierendes Zielgas vorhanden ist. Der Vorteil eines geringen Energieverbrauchs ist insbesondere dann relevant, wenn der erfindungsgemäße Sensor als ein tragbares Gerät verwendet wird und eine eigene Spannungsversorgungseinheit umfasst.

Zu untersuchendes Gas kann durch den messzellenseitigen Einlass hindurch in den Innenraum und dort zur Messzelle diffundieren oder anderweitig fließen, beispielsweise angesaugt werden. Die chemische Reaktion an der Messzelle kann zu einem Überdruck im Innenraum führen. Dank des Druckausgleichs-Auslasses kann dieser Überdruck in die Umgebung abgebaut werden. In der Regel ist keine Fluidfördereinheit erforderlich, um diesen Überdruck abzubauen.

Jedoch kann insbesondere dann, wenn im Innenraum kein Überdruck herrscht, zu untersuchendes oder sonstiges Gas auch durch den Druckausgleichs-Auslass hindurch in den Innenraum gelangen. Wenn dieses Gas die Messzelle erreicht, also quasi von der anderen Seite, kann dieses Gas eine Messung der Messzelle verfälschen. Ein wesentlicher Grund für eine mögliche Verfälschung ist die, dass das Gas eine Referenzspannung der Messelektrode verändert, wobei die beeinflusste elektrische Größe von dieser Referenzspannung abhängt. Diese Referenzspannung würde insbesondere dann verfälscht, wenn die Referenzspannung von einer optionalen Referenzelektrode festgelegt wird und das Gas durch den Druckausgleichs-Auslass hindurch die Referenzelektrode erreicht. Insbesondere aus diesem Grund sollte zu untersuchendes Gas nicht durch den Druckausgleichs-Auslass hindurch die Messzelle erreichen, sondern nur durch den messzellenseitigen Einlass hindurch. Außerdem könnte Gas, welches durch den Druckausgleichs-Auslass hindurch in den Innenraum gelangt, zu Ablagerungen auf einer Elektrode der Messzelle führen.

Das erfindungsgemäße Oxidierungs-Bauteil schafft eine Lösung für dieses Problem. Das Oxidierungs-Bauteil ist in einer Fluidverbindung zwischen dem Druckausgleichs-Auslass und der Messzelle angeordnet und oxidiert alle oxidierbaren Bestandteile in einem Gas, welches durch den Druckausgleichs-Auslass hindurch in den Innenraum fließt. Dadurch verhindert das Oxidierungs-Bauteil vollständig oder wenigstens weitgehend, dass ein oxidierbarer Gas-Bestandteil vom Druckausgleichs-Auslass her die Messzelle erreicht. Andererseits nimmt das Oxidierungs-Bauteil idealerweise keinen Einfluss auf ein Gas, welches durch den messzellenseitigen Eingang hindurch in den Innenraum fließt und die Messzelle erreicht.

Möglich, aber dank des Oxidierungs-Bauteils nicht erforderlich ist es, den Druckausgleichs-Auslass mit einem mit einem für Gas undurchlässigen Verschluss zu verschließen, solange kein Überdruck im Innenraum vorhanden ist, ist, und diesen Verschluss nur zum Abbau eines Überdrucks zu entfernen. Ein solcher Verschluss könnte sich versehentlich auch dann auf dem Druckausgleichs-Auslass befinden, wenn ein Überdruck vorhanden ist und abgebaut werden sollte. Möglich ist, den Druckausgleichs-Auslass mit einem für Gas durchlässigen Verschluss zu verschließen, wobei Überdruck durch diesen Verschluss hindurch abgebaut werden kann und wobei dieser Verschluss den Innenraum bis zu einem gewissen Grad vor mechanischen Beschädigungen von außen schützt. Möglich ist, den Druckausgleichs-Auslass dann mit einem für Gas undurchlässigen Verschluss zu verschließen, wenn der elektrochemische Sensor nicht benutzt wird.

Erfindungsgemäß ist das Oxidierungs-Bauteil zwischen dem Druckausgleichs-Auslass und der Messzelle angeordnet. Weil das Oxidierungs-Bauteil oxidierbare Gas-Bestandteile oxidiert und mit einem Abstand zur Messzelle angeordnet ist, ist die Gefahr geringer, dass ein oxidierbarer Gas-Bestandteil eine Referenzspannung der Messelektrode verändert und dadurch ein Messergebnis verfälscht, verglichen mit einer Ausgestaltung ohne Oxidierungs-Bauteil und / oder ohne Abstand. Der Abstand ermöglicht es, mindestens eine Sperrschicht zwischen dem Oxidierungs-Bauteil und der Messzelle anzuordnen.

An das Oxidierungs-Bauteil lässt sich ein ausreichend hohes elektrisches Potenzial anlegen. Dieses hohe elektrische Potenzial erhöht die Sicherheit, dass alle oxidierbaren Bestandteile im Innenraum tatsächlich oxidiert werden und keine relevante Menge eines oxidierbaren Bestandteils die Messzelle erreicht. Weil das Oxidierungs-Bauteil elektrisch arbeitet, verbraucht es sich nicht oder wenigstens langsamer als andere mögliche Bauteile, welche ein Gas zu oxidieren vermögen. Insbesondere wird ermöglicht, dass das Oxidierungs-Bauteil keine chemische Substanz aufzuweisen braucht, wobei diese Substanz de sich im Laufe des Einsatzes verbraucht.

Weil bevorzugt das Oxidierungs-Bauteil mit der Messzelle ionisch leitend verbunden ist, vermag die Messzelle ein elektrisches Referenzpotenzial und / oder eine elektrische Referenzspannung des Oxidierungs-Bauteils festzulegen.

Das Oxidierungs-Bauteil weist bevorzugt ein elektrisches Potenzial auf, das oberhalb des elektrischen Potenzials der Messelektrode liegt. Dadurch wird besonders zuverlässig und rasch verhindert, dass ein oxidierbarer Gas-Bestandteil durch den Druckausgleichs-Auslass hindurch zur Messzelle gelangt, insbesondere organische Dämpfe. Dieser Gas-Bestandteil wird vielmehr zuverlässig durch das Oxidierungs-Bauteil oxidiert.

Insbesondere dann, wenn der erfindungsgemäße Sensor in Umgebungsluft eingesetzt wird, kann die Messzelle sich stark erhitzen und könnte sogar beschädigt werden, wenn viel Sauerstoff die Messzelle erreicht. Bekanntlich weist Umgebungsluft einen Sauerstoffanteil von etwa 20 % auf. **In** einer bevorzugten Ausgestaltung ist daher ein Volumenfluss-Reduzierer zwischen dem messzellenseitigen Einlass und der Messzelle angeordnet. Dieser Volumenfluss-Reduzierer ist bevorzugt ein mechanischer Filter und reduziert den Volumenfluss eines Gases zur Messzelle, wobei dieses Gas durch den messzellenseitigen Einlass hindurch in den Innenraum fließt, sehr stark verglichen mit einem Zustand, bei dem kein Volumenfluss-Reduzierer zwischen dem messzellenseitigen Einlass und der Messzelle angeordnet ist. "Sehr stark" bedeutet: um mindestens 95 %, bevorzugt um mindestens 99 %, besonders bevorzugt um mindestens 99,9 %, insbesondere um mindestens 99,99 %.

Der Volumenfluss-Reduzierer umfasst bevorzugt mindestens eine Sperrschicht. Diese Sperrschicht hat bevorzugt eine Dicke von weniger als 0,1 mm, besonders bevorzugt weniger als 20 µm. Dank des Oxidierungs-Bauteils braucht zwischen dem Druckausgleichs-Auslass und der Messzelle kein solcher Volumenfluss-Reduzierer angeordnet zu sein. Daher wird ein möglicher Überdruck im Innenraum schneller abgebaut, als wenn auch der Volumenfluss durch den Druckausgleichs-Auslass hindurch durch einen weiteren Volumenfluss-Reduzierer reduziert werden würde.

**In** einer bevorzugten Ausgestaltung umfasst der elektrochemische Sensor eine Referenzelektrode (Bezugselektrode). Diese Referenzelektrode lässt sich als ein Bestandteil der Messzelle bezeichnen. Diese Referenzelektrode weist ein konstantes elektrisches Potenzial auf und legt dadurch die elektrische Referenzspannung der Messelektrode fest. Bevorzugt ist die Referenzelektrode mit der Messelektrode ionisch leitend verbunden und legt dadurch das elektrische Potenzial der Messelektrode fest.

Bevorzugt ist das Oxidierungs-Bauteil zwischen der Referenzelektrode und dem Druckausgleichs-Auslass angeordnet. Besonders bevorzugt tritt zwischen der Referenzelektrode und dem Oxidierungs-Bauteil ein Abstand auf. Diese Positionierung reduziert weiter die Gefahr, dass eine relevante Menge eines brennbaren Gas-Bestandteils durch den Druckausgleichs-Auslass hindurch die Referenzelektrode erreicht Referenzelektrode erreicht und in unerwünschter Weise die Referenzspannung verändert. Der Abstand ermöglicht es, mindestens eine Sperrschicht vorzusehen.

Das Oxidierungs-Bauteil umfasst mindestens eine Elektrode bevorzugt zwei Elektroden. Bevorzugt ist das Oxidierungs-Bauteil nicht nur mit der Messzelle, sondern zusätzlich mit der Referenzelektrode ionisch leitend verbunden. Dadurch legt die Referenzelektrode auch das elektrische Potenzial der oder mindestens einer Elektrode des Oxidierungs-Bauteils fest.

**In** einer Realisierungsform umfasst das Oxidierungs-Bauteil zwei Elektroden und eine Schicht zwischen den beiden Elektroden, wobei diese Schicht einerseits die beiden Elektroden elektrisch voneinander isoliert und andererseits die beiden Elektroden ionisch leitend miteinander verbindet. Diese Schicht kann so wie die Schicht zwischen den beiden Elektroden der Messzelle aufgebaut sein und insbesondere einen Elektrolyten umfassen.

Der Druckausgleichs-Auslass lässt sich auch als ein Einlass verwenden, verwenden, und zwar insbesondere vor einem Einsatz des Sensors. Bevorzugt kann ein flüssiger Elektrolyt in das Gehäuse und dort durch den Druckausgleichs-Auslass hindurch und am Oxidierungs-Bauteil vorbei zur Messzelle fließen. Diese Ausgestaltung ermöglicht es, vor einem Einsatz den elektrochemischen Sensor mit flüssigem Elektrolyten zu befüllen und hierbei insbesondere zu erreichen, dass der eingefüllte flüssige Elektrolyt das Oxidierungs-Bauteil und die Messzelle erreicht und die erforderlichen oder gewünschten ionisch leitenden Verbindungen herstellt. Bevorzugt wird der Sensor in eine Position verbracht, in der sich die Messzelle senkrecht oder schräg unterhalb des Druckausgleichs-Auslasses befindet. Die Schwerkraft bewirkt, dass der Elektrolyt im Innenraum senkrecht oder schräg nach unten bis zur Messzelle fließt. Idealerweise ist es nicht erforderlich, nach der Inbetriebnahme des Sensors flüssiges Elektrolyt nachzufüllen.

**In** einer Ausgestaltung ist zwischen dem Druckausgleichs-Auslass und der Messzelle mindestens eine für Gas undurchlässige Sperrschicht angeordnet. In die oder jede Sperrschicht ist jeweils mindestens eine für Gas durchlässige Durchgangsöffnung eingelassen, optional in einer Sperrschicht mindestens zwei durchlässige Durchgangsöffnungen. Die Ausgestaltung mit der Sperrschicht reduziert weiter die Gefahr, dass Gas, das durch den Druckausgleichs-Auslass hindurch in den Innenraum geflossen ist, die Messzelle erreicht. Dank der Durchgangsöffnung kann trotzdem ein Druckausgleich stattfinden.

Erfindungsgemäß kann ein Gas aus der Umgebung durch den messzellenseitigen Einlass hindurch in den Innenraum fließen. In einer Ausgestaltung fließt das Gas aus einem räumlichen Bereich, der direkt an das Gehäuse des elektrochemischen Sensors angrenzt, in den Innenraum, beispielsweise durch Diffusion und / oder durch Ansaugung. In einer Ausgestaltung lässt sich auf den messzellenseitigen Einlass ein Adapter aufsetzen und bevorzugt wieder abnehmen. Dieser Adapter ist fluiddicht mit einem Ende einer Fluidführungseinheit verbunden, wobei diese Fluidführungseinheit außerhalb des elektrochemischen Sensors angeordnet ist. Bei aufgesetztem Adapter ist der messzellenseitige Einlass nur durch die Fluidführungseinheit mit der Umgebung verbunden. Mit anderen Worten: Eine Probe eines zu untersuchenden Gases kann nicht den Adapter umgehen, um in den Innenraum des Sensors zu gelangen. Das Gas fließt durch diese Fluidführungseinheit und durch den messzellenseitigen Einlass hindurch in den Innenraum. Der Adapter überdeckt bevorzugt nicht den Druckausgleichs-Auslass, so dass auch bei aufgesetzten Adapter ein Überdruck im Innenraum rasch abgebaut werden kann. Bevorzugt lässt sich der Sensor wahlweise mit aufgesetztem Adapter oder ohne Adapter verwenden. Bevorzugt umfasst der Sensor oder auch der Adapter eine Fluidfördereinheit, welche ein Gas durch die Fluidführungseinheit hindurch anzusaugen vermag.

Unter einer Fluidführungseinheit wird ein Bauteil verstanden, welches ein Fluid entlang einer vorgegebenen Trajektorie zu führen vermag und idealerweise verhindert, dass das Fluid diese Trajektorie verlässt. Ein Schlauch und eine Röhre sind zwei Beispiele für eine Fluidführungseinheit. Eine Fluidfördereinheit vermag einen Fluss von Fluid zu bewirken. Eine Pumpe, ein Gebläse und eine Kolben-Zylinder-Einheit sind drei Beispiele für eine Fluidfördereinheit.

Die Ausgestaltung mit dem aufsetzbaren Adapter und der Fluidführungseinheit ermöglicht es, ein Gas in einem räumlichen Bereich zu untersuchen, der räumlich vom elektrochemischen Sensor beabstandet ist. Dieser beabstandete Bereich kann insbesondere ein umschlossener Raum sein, und der elektrochemische Sensor lässt sich außerhalb dieses Raums anordnen. Substanzen im umschlossenen Raum können nur durch die Fluidführungseinheit und den messzellenseitigen Eingang hindurch den Innenraum des Sensors erreichen, aber nicht durch den Druckausgleichs-Auslass.

Der erfindungsgemäße Sensor kann als ein tragbares Gerät ausgestaltet sein und eine eigene Spannungsversorgungseinheit aufweisen. Ein Benutzer kann den erfindungsgemäßen Sensor mit sich führen, um ein Gas in seiner Umgebung zu untersuchen. Der erfindungsgemäße Sensor kann auch als ein ortsfestes Gerät ausgestaltet sein und sich mit einem stationären Spannungsversorgungsnetz verbinden lassen. Optional umfasst der erfindungsgemäße Sensor eine Fluidfördereinheit, z.B. eine Pumpe oder ein Gebläse, welche(s) Gas aus der Umgebung anzusaugen vermag. Der erfindungsgemäße Sensor kann eine eigene Ausgabeeinheit umfassen, welche ein Messergebnis der Messzelle in einer von einem Menschen wahrnehmbaren Weise ausgibt. Der Sensor kann eine eigene Alarmeinheit umfassen, welche einen Alarm in einer von einem Menschen wahrnehmbaren Form ausgibt, wenn ein Messwert außerhalb eines vorgegebenen Bereichs liegt. Bevorzugt wird der Alarm in einer taktil wahrnehmbaren Form ausgegeben, beispielsweise durch Vibrationen. Möglich ist auch, dass der erfindungsgemäße Sensor eine Kommunikationseinheit umfasst, welche ein Messergebnis oder einen Alarm der Messzelle an einen räumlich entfernten Empfänger zu übermitteln vermag.

Im Folgenden wird die Erfindung anhand eines Ausführungsbeispiels beschrieben. Hierbei zeigt
- Figur 1: schematisch in einer Querschnittsdarstellung, wie Gas in das Innere des Sensors gelangen kann;
- Figur 2: schematisch in einer Querschnittsdarstellung den Aufbau des erfindungsgemäßen Sensors;.
- Figur 3: schematisch in der Querschnittsdarstellung von Figur 1 einen Adapter und einen Schlauch auf dem messzellenseitigen Einlass.

Im Ausführungsbeispiel wird der erfindungsgemäße Sensor dafür verwendet, um zu detektieren, ob ein Gas einen vorgegebenen Gas-Bestandteil aufweist oder nicht, und / oder um den Gehalt (die Konzentration) dieses Gas-Bestandteils in dem Gas zu messen. Der Gas-Bestandteil ist beispielsweise Sauerstoff in Form von O₂ oder ein Gasgemisch, welches Sauerstoff enthält, beispielsweise N₂O oder H₂O oder CO, oder Wasserstoff in Form von H₂ oder organische Dämpfe. In der nachfolgenden Beschreibung wird beispielhaft Sauerstoff als der zu detektierende Gas-Bestandteil genannt. In dem zu untersuchenden Gas können zusätzlich zu dem zu detektierenden Sauerstoff oxidierbare Gas-Bestandteile vorhanden sein, beispielsweise SO₂, NO₂, Alkohol, Aldehyde und / oder ungesättigte Kohlenwasserstoffe.

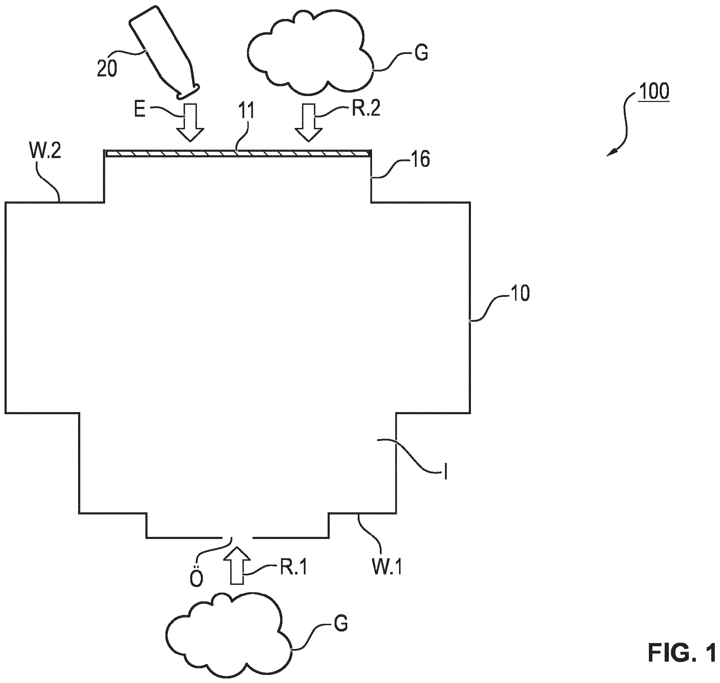

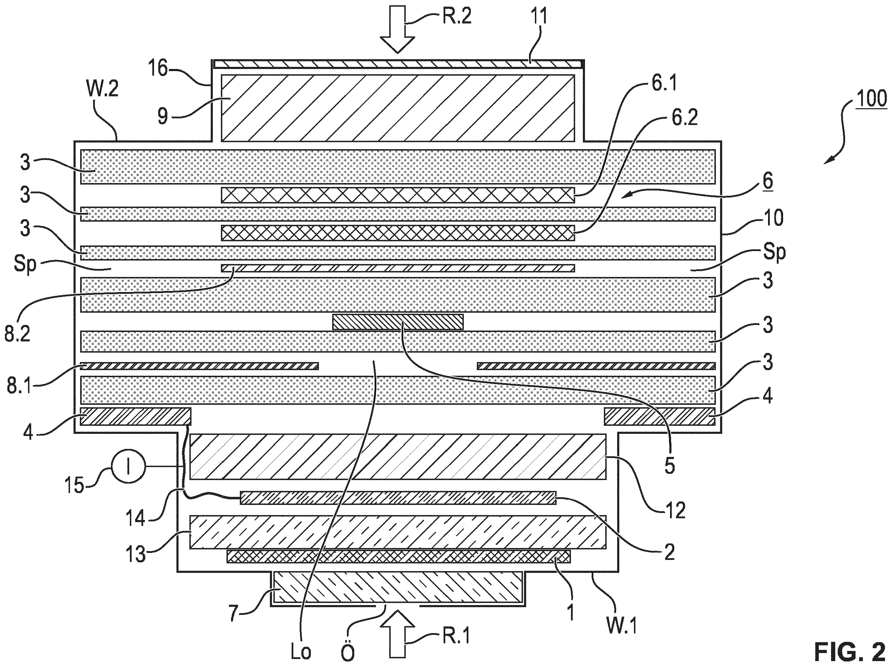

Figur 1 zeigt schematisch in einer Querschnittsdarstellung, wie Gas G in das Innere eines erfindungsgemäßen elektrochemischen Sensors 100 gelangen kann. Figur 2 zeigt schematisch einen Querschnitt durch den erfindungsgemäßen Sensor 100 von Figur 1.

Der Sensor 100 umfasst ein Gehäuse 10 mit einer Abdeckung 11, wobei das Gehäuse 10 einen Innenraum I umgibt. In einer Ebene, die senkrecht auf den Zeichenebenen von Figur 1 und Figur 2 steht, hat das Gehäuse 10 bevorzugt eine kreisrunde Querschnittsfläche.

Beim Einsatz des Sensors 100 strömt ein zu untersuchendes Gas G in die Richtung R.1 durch eine Öffnung Ö in den Innenraum I, in Figur 1 und Figur 2 also nach oben. Diese Öffnung wird nachfolgend als die messzellenseitige Öffnung bezeichnet und fungiert als der messzellenseitige Einlass des Ausführungsbeispiels. Die Öffnung ist in eine Wand W.1 des Gehäuses 10 eingelassen.

Bei der Fertigstellung des Sensors 100 und / oder vor einem Einsatz wird ein flüssiger Elektrolyt E aus einem Behälter 20 in den Innenraum I verbracht. Der Elektrolyt E strömt hierbei im Beispiel von Figur 1 von oben in den Innenraum I hinein. Der flüssige Elektrolyt E wird beispielsweise bei abgenommener Abdeckung 11 oder durch eine nicht gezeigte Öffnung in der Abdeckung 11 hindurch in den Innenraum I eingefüllt. Als Elektrolyt E wird beispielsweise eine wässrige Lösung umfassend Schwefelsäure oder Phosphorsäure oder Perchlorsäure mit einem Wasseranteil von mindestens 5 % verwendet. Der Siedepunkt des flüssigen Elektrolyten E liegt oberhalb der Temperatur, bei der der Sensor 100 eingesetzt wird, damit der Elektrolyt E nicht verdampft.

Im Innenraum I sind eine Messelektrode (Arbeitselektrode) 2 und eine Gegenelektrode 4 angeordnet, vgl. Figur 2. Im gezeigten Beispiel hat die Messelektrode 2 die Form einer Scheibe, die Gegenelektrode 4 die Form eines Kreisrings (Torus). In einer Ausgestaltung finden folgende chemische Reaktionen statt:
- Sauerstoff auf der Messelektrode 2 wird zu 2O²⁻ reduziert. Beispielsweise findet die chemische Reaktion O₂ + 2H₂O + 4e⁻ → 4OH⁻ statt.
- 2O²⁻ , das im Elektrolyten E enthalten ist, wird an der Gegenelektrode 4 zu O₂ oxidiert. Beispielsweise findet die umgekehrte chemische Reaktion 2H₂O → O₂ + 4H⁺ + 4e⁻ statt.

Eine Referenzelektrode (Bezugselektrode) 5 legt das elektrische Potenzial an der Messelektrode 2 fest. Bevorzugt liegt das elektrische Potenzial der Messelektrode 2 um mehrere 100 mV unter dem der Referenzelektrode 5. Dadurch wird die chemische Reaktion an der Messelektrode 2 bewirkt. Insbesondere bewirkt das elektrische Potenzial der Messelektrode 2 die oben beschriebenen chemischen Reaktionen. Idealerweise wird die folgende Situation bewirkt: Die Konzentration von O₂ an der Messelektrode 2 beträgt aufgrund der chemischen Reaktion dauerhaft null. An der Gegenelektrode 4 wird hingegen O₂ erzeugt. Eine Realisierungsform einer solchen Messzelle lässt sich als "Sauerstoffpumpe" bezeichnen.

Zwischen den beiden Elektroden 2 und 4 bildet sich durch die chemischen Reaktionen ein O₂-Konzentrations-Gradient. Dieser O₂-Gradient führt dazu, dass Elektronen von der Gegenelektrode 4 zu der Messelektrode 2 fließen. Daher fließt ein elektrischer Strom durch einen Draht 14, der die Messelektrode 2 mit der Gegenelektrode 4 verbindet und beabstandet vom Elektrolyten 12 angeordnet ist. Die beiden Elektroden 2 und 4, der Elektrolyt 12 und der Draht 14 bilden zusammen einen Stromkreis. In dem Draht 14 ist ein nicht gezeigter elektrischer Messwiderstand angeordnet. Eine Messeinheit in Form eines Stromstärken-Sensors 15 misst die Stärke oder auch die Menge (elektrische Ladung) des Stroms, der durch den Draht 14 fließt. Die Stromstärke oder auch die elektrische Ladung sind ein Maß für die Konzentration bzw. den Gehalt von Sauerstoff im Innenraum I im Sensor 100.

Die beiden Elektroden 2 und 4, die Isolierschicht 12, der Draht 14 und der Stromstärken-Sensor 15 gehören im Ausführungsbeispiel zur Messzelle des Sensors 100.

Der Abstand zwischen den beiden Elektroden 2 und 4 ist so groß, und die Isolierschicht 12 mit dem Elektrolyten E ist so ausgestaltet, dass folgendes bewirkt wird: Weitgehend verhindert wird, dass O₂ zurück von der Gegenelektrode 4 zur Messelektrode 2 diffundiert. Die Elektroden 2 und 4 sind aus einem Metall hergestellt, bevorzugt einem Edelmetall, welches resistent gegen Materialien des Elektrolyten E sowie gegen CO, H₂S und andere mögliche Bestandteile des Gases G ist. Besonders bevorzugt sind die beiden Elektroden 2, 4 aus Platin oder aus Gold hergestellt. Bei diesem Material finden die oben beschriebenen chemischen Reaktionen ausreichend rasch statt. Andererseits ist dieses Material chemisch resistent gegen den Elektrolyten E. Möglich ist auch, dass die Messelektrode 2 und / oder die Gegenelektrode 4 aus Gold hergestellt sind.

Die beiden Elektroden 2, 4 sind durch eine Isolierschicht 12 mit mindestens einer Lage aus Glasfaser oder einem Glasvlies, die mit dem Elektrolyten E getränkt ist, voneinander elektrisch isoliert. Zugleich ermöglicht der Elektrolyt E den Fluss von Ionen.

Die Abdeckung 11 ist auf eine Stützhülse 16 aufgesetzt, die mechanisch mit einer Wand W.2 des Gehäuses 10 verbunden ist. Die von dieser Stützhülse 16 umschlossene Fläche fungiert als der Druckausgleichs-Auslass des Ausführungsbeispiels. Im Inneren dieser Stützhülse 16 ist ein elastischer gasdurchlässiger Stempel 9 angeordnet. Dieser Stempel 9 drückt die Elektroden und sonstigen Schichten zusammen und bewirkt, dass die Elektroden eine nur relativ geringe Relativbewegung zueinander ausführen können und daher der Draht 14 dauerhaft und zuverlässig die beiden Elektroden 2 und 4 kontaktiert.

Bekanntlich weist Luft einen Sauerstoffgehalt von etwa 20 % auf. Eine so große Menge von Sauerstoff würde zu einem großen Umsatz von Sauerstoff an der Messelektrode 2 führen und die Messelektrode 2 rasch beschädigen. Daher trennt ein Volumenfluss-Reduzierer in Form einer dünnen Sperrschicht 1 die Messelektrode 2 beinahe fluiddicht von der Umgebung. "Beinahe fluiddicht" bedeutet im Ausführungsbeispiel, dass weniger als 1 %, insbesondere weniger als 1 ‰, bevorzugt weniger als 0,1 ‰, des Gases G, welches aus der Umgebung in die Richtung R.1 durch die Öffnung Ö in den Innenraum I fließt, die Sperrschicht 1 durchdringen und die Messelektrode 2 erreichen kann. Diese geringe Menge reicht aus, damit die gemessene Stromstärke oder elektrische Ladung durch den Draht 14 mit ausreichender Zuverlässigkeit mit der gesuchten Sauerstoff-Konzentration korreliert.

Die Sperrschicht 1 hat im Ausführungsbeispiel eine Dicke von mindestens 5 µm und weniger als 50 µm, bevorzugt weniger als 20 µm, besonders bevorzugt eine Dicke zwischen 10 µm und 15 µm. Verhindert werden soll, dass ein Riss oder Loch in der Sperrschicht 1 auftritt. Im gezeigten Beispiel ist die Sperrschicht 1 nach Art eines Sandwichs zwischen zwei Stützmembranen 7 und 13 angeordnet. Jede Stützmembrane 7, 13 ist beispielsweise aus PTFE aufgebaut. Die Stützmembrane 7 zwischen der Öffnung Ö und der Sperrschicht 1 schützt die Sperrschicht 1 vor mechanischen Beschädigungen aufgrund von Einflüssen, die von außen durch die Öffnung Ö einwirken. Die Stützmembrane 13 zwischen der Sperrschicht 1 und der Messelektrode 2 schützt die Sperrschicht 1 vor einer möglichen mechanischen Beschädigung durch den Draht 14.

Gesehen in die Strömungsrichtung R.1 flussabwärts von der Gegenelektrode 4 ist die Referenzelektrode (Bezugselektrode) 5 angeordnet. Diese Referenzelektrode 5 liefert ein elektrisches Referenzpotenzial und verhindert, dass bei gleichbleibender Sauerstoff-Konzentration die Stromstärke oder die elektrische Ladung, welche der Stromstärken-Sensor 15 misst, driftet oder oszilliert. Weil die Referenzelektrode 5 mit einem ausreichend großen Abstand flussabwärts von den beiden Elektroden 2 und 4 angeordnet ist, liegt die Referenzelektrode 5 außerhalb des Bereichs, in dem der oben beschriebene O₂-Konzentrations-Gradient auftritt. Daher wirkt dieser O₂-Gradient nicht auf die Referenzelektrode 5 ein und beeinflusst idealerweise nicht die Referenzspannung, welche die Referenzelektrode 5 festlegt.

Im Innenraum I sind mehrere parallele Isolierschichten 3 angeordnet, die mit dem Elektrolyten E getränkt sind und beispielsweise Glasvlies umfassen. Durch die Isolierschicht 3 hindurch erreicht der Elektrolyt E die Isolierschicht 12 und benetzt die Oberfläche der Messelektrode 2. Weil die Gegenelektrode 4 die Form eines Kreisrings aufweist, kann der Elektrolyt E durch das Loch im Kreisring zur Messelektrode 2 fließen. Die Membrane 13 zwischen der Messelektrode 2 und der Sperrschicht 1 verhindert, dass der Elektrolyt E weiter in Richtung der Sperrschicht 1 fließt.

In einer Ausgestaltung wird die Referenzelektrode 5 von beiden Seiten nach Art eines Sandwichs von jeweils einer Isolierschicht 3 mit Elektrolyten E umgeben. Zwischen der Referenzelektrode 5 und der Gegenelektrode 4 ist mindestens eine Isolierschicht 3 angeordnet, im gezeigten Beispiel sogar zwei Isolierschichten 3. Dadurch ist die Referenzelektrode 5 elektrisch von der Gegenelektrode 4 isoliert, aber ionisch leitend verbunden. Die Referenzelektrode 5 wird aufgrund des oben beschriebenen Abstands und dieser elektrischen Isolierung nicht in nennenswerter Weise vom O₂-Gradienten zwischen den beiden Elektroden 2 und 4 beeinflusst. Dieser unerwünschte Effekt wird weitgehend verhindert, weil die Isolierschichten 3 elektrisch isolierend wirken und weil Sauerstoff relativ langsam diffundiert.

Im Innenraum I kann durch eine chemische Reaktion ein Überdruck entstehen, insbesondere durch eine der gerade beschriebenen Reaktionen an der Messzelle. Dieser Überdruck muss rasch abgebaut werden, insbesondere weil der Überdruck dazu führen kann, dass Sauerstoff im Elektrolyten E sich löst und zur Messelektrode 2 diffundiert, was falsche Messergebnisse zur Folge haben kann. Daher ist in einer Ausgestaltung die optionale Abdeckung 11 gasdurchlässig oder weist dauerhaft eine Öffnung auf. Auch der Stempel 9 ist gasdurchlässig. Die Abdeckung 11 schützt den Stempel 9 vor mechanischen Beschädigungen von außen. Der Überdruck im Innenraum I kann daher durch den Stempel 9 und die Abdeckung 11 hindurch abgebaut werden. Der Stempel 9 und die Abdeckung 11 sind im bzw. auf dem Druckausgleichs-Auslass 16 des Ausführungsbeispiels angeordnet.

Wie oben dargelegt, wird an der Gegenelektrode 4 Sauerstoff in Form von O₂ erzeugt. Die Sperrschicht 12 mit dem Elektrolyten E verhindert das unerwünschte Ereignis, dass dieser Sauerstoff an die Messelektrode 2 gelangt. Insbesondere dank des Sauerstoffs, der an der Gegenelektrode 4 erzeugt wird und nicht die Sperrschicht 12 durchdringen kann, tritt im Bereich zwischen der Gegenelektrode 4 und der Abdeckung 11 ein Überdruck auf. Im Ausführungsbeispiel ist die Gegenelektrode 4 als ein Ring ausgestaltet, und zwischen der Referenzelektrode 5 und dem Gehäuse 10 tritt ein Abstand auf. Diese Realisierungsform erleichtert das gewünschte Ergebnis, dass dieser Überdruck in Richtung R.1 durch den Druckausgleichs-Auslass 16 hindurch abgebaut wird, in Figur 1 und Figur 2 also nach oben.

Bei entfernter Abdeckung 11 oder durch die Abdeckung 11 oder durch eine Öffnung in der Abdeckung 11 sowie durch den elastischen Stempel 9 hindurch kann Gas G aus der Umgebung in die Richtung R.2 fließen, in Figur 1 und Figur 2 also nach unten, und dadurch in den Innenraum I gelangen. Dies gilt insbesondere dann, wenn im Innenraum I kein Überdruck relativ zur Umgebung auftritt. Dieses Gas G kann mindestens einen oxidierbaren Gas-Bestandteil aufweisen, der das Referenzpotential der Referenzelektrode 5 verschieben oder der zu Ablagerungen auf einer Elektrode 2, 4, 5 führen kann. Beispiele für derartige oxidierbare Gas-Bestandteile wurden eingangs genannt.

Zwischen dem Stempel 9 einerseits und den Elektroden 5, 4, 2 andererseits ist ein Oxidierungs-Bauteil 6 angeordnet. Dieses Oxidierungs-Bauteil 6 verhindert weitgehend, dass oxidierbare Gas-Bestandteile, die durch den Druckausgleichs-Auslass 16 hindurch in den Innenraum I geflossen sind, eine Elektrode 5, 4, 2 erreichen. Das Oxidierungs-Bauteil 6 umfasst mindestens eine Elektrode, im gezeigten Beispiel zwei voneinander beabstandete Elektroden 6.1 und 6.2. In einer Ausgestaltung umfasst das Oxidierungs-Bauteil 6 genau wie die Messzelle zwei Elektroden 6.1, 6.2 sowie eine ionisch leitende Verbindung zwischen den Elektroden 6.1 und 6.2, beispielsweise eine mit dem Elektrolyten E gesättigte Schicht 3. Auch zwischen diesen beiden Elektroden 6.1 und 6.2 tritt ein O₂-Gradient auf.

Bevorzugt ist die oder jede Elektrode 6.1, 6.2 des Oxidierungs-Bauteils 6 aus Platin oder Gold. Die oder mindestens eine Elektrode 6.1, 6.2 des Oxidierungs-Bauteils 6 wird auf einem elektrischen Potenzial gehalten, das von dem elektrischen Potenzial der Referenzelektrode 5 abweicht und zwischen 0,1 V und 0,5 V, bevorzugt zwischen 0,2 V und 0,4 V liegt. Das Oxidierungs-Bauteil 6 oxidiert daher alle oxidierbaren Bestandteile des Gases G, welche durch die Abdeckung 11 und den Stempel 9 hindurch in die Richtung R.2 in den Innenraum I diffundieren, und zwar idealerweise vollständig.

Mindestens eine Isolierschicht 3 isoliert das Oxidierungs-Bauteil 6 elektrisch von der Referenzelektrode 5, im gezeigten Beispiel zwei voneinander beabstandete Isolierschichten 3. Das Oxidierungs-Bauteil 6 ist ionisch leitend mit der Referenzelektrode 5 verbunden, im Ausführungsbeispiel durch den Elektrolyten E in der Isolierschicht 3. Dank der ionisch leitenden Verbindung legt die Referenzelektrode 5 zusätzlich das elektrische Potenzial des Oxidierungs-Bauteils 6 oder wenigstens das elektrische Potenzial einer Elektrode 6.1 oder 6.2 des Oxidierungs-Bauteils 6 fest.

Optional sind zwischen dem Oxidierungs-Bauteil 6 und der Gegenelektrode 4 zwei gasundurchlässige Sperrfolien 8.1 und 8.2 angeordnet. Die beiden Sperrfolien 8.1 und 8.2 sind beispielsweise aus einem für Gas undurchlässigen Kunststoff hergestellt, beispielsweise aus einem Perfluoralkoxy-Polymer (PFA) oder einem Polyvinylidenfluorid (PVDF). Die Sperrfolien 8.1 und 8.2 reduzieren das Risiko, dass O₂ von der Gegenelektrode 4 zur Referenzelektrode 5 oder gar zum Oxidierungs-Bauteil 6 diffundiert oder dass ein Gas, das in die Richtung R.2 fließt, eine Elektrode 2, 4 erreicht. Die beiden Sperrfolien 8.1 und 8.2 sind resistent gegen den Elektrolyten E. Damit der Elektrolyt E trotzdem die Messelektrode 2 erreichen kann, tritt zwischen der Sperrfolie 8.2 und dem Gehäuse 10 ein ausreichend großer Spalt Sp auf. Die Sperrfolie 8.1 hat die Form eines Rings, sodass der Elektrolyt E durch das Loch Lo in diesem Ring hindurch zur Messelektrode 2 fließen kann.

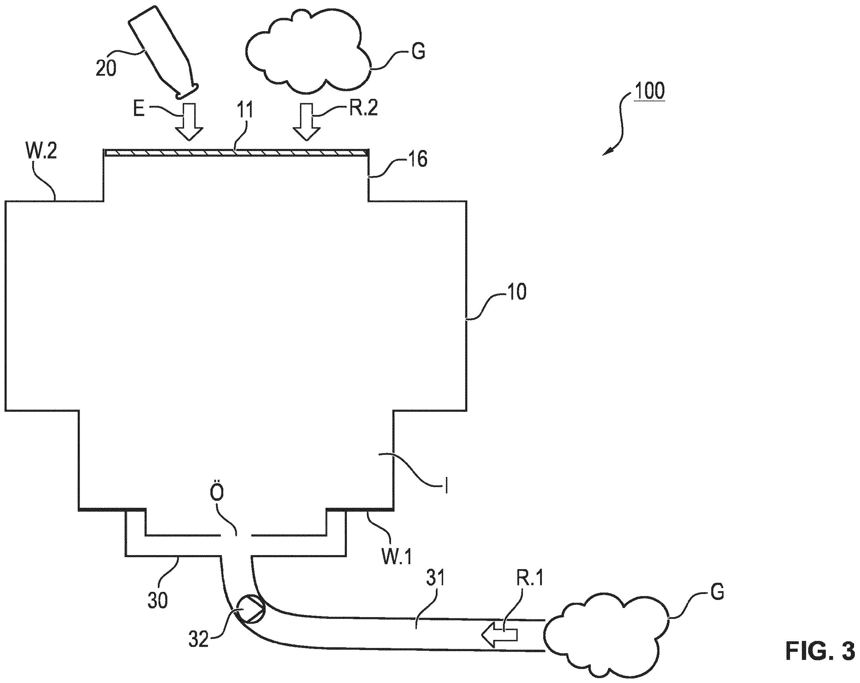

In der Ausgestaltung gemäß Figur 1 diffundiert das Gas G aus einem räumlichen Bereich, der unmittelbar an die Öffnung Ö angrenzt, in den Innenraum I. Figur 3 zeigt eine alternative Ausgestaltung. Auf die Öffnung Ö ist ein Adapter 30 aufgesetzt. Der Adapter 30 ist fluiddicht mit einem Schlauch 31 verbunden. Das Gas G kann nur durch den Schlauch 31 hindurch die Öffnung Ö erreichen, aber nicht am Adapter 30 vorbei. Abgesehen vom Schlauch 31 umgibt der Adapter 30 nämlich die Öffnung Ö fluiddicht. Das freie Ende des Schlauchs 31 befindet sich in einem Bereich mit zu untersuchendem Gas. Eine schematisch gezeigte Pumpe 32 saugt das Gas G durch den Schlauch 31 hindurch an und leitet es weiter durch die Öffnung Ö in den Innenraum I. Genau wie eine Ausgestaltung der Figur 1 kann Gas G auch durch den Druckausgleichs-Auslass 16 hindurch den Innenraum I erreichen.

Der Adapter 30 lässt sich wieder von der Öffnung Ö abnehmen, so das Gas G dann wieder direkt durch die Öffnung Ö hindurch den Innenraum erreicht, so wie dies in Figur 1 angedeutet wird.

**Bezugszeichenliste**

| | |
|---|---|
| 1 | einlassseitige Sperrschicht, zwischen den Stützmembranen 7 und 13 angeordnet, fungiert als Volumenfluss-Reduzierer für die Öffnung Ö |
| 2 | Messelektrode, gehört zur Messzelle |
| 3 | Isolierschicht, mit dem Elektrolyten E getränkt |
| 4 | ringförmige Gegenelektrode, gehört zur Messzelle |
| 5 | Referenzelektrode (Bezugselektrode), gehört zur Messzelle |
| 6 | Oxidierungs-Bauteil, umfasst mindestens eine Elektrode, bevorzugt die beiden Elektroden 6.1 und 6.2, zwischen dem Druckausgleichs-Auslass 16 und der Messzelle angeordnet |
| 6.1, 6.2 | Elektroden des Oxidierungs-Bauteils 6 |
| 7 | gasdurchlässige Stützmembrane zwischen der Sperrschicht 1 und der Öffnung Ö |
| 8.1 | ringförmige Sperrfolie zwischen der Referenzelektrode 5 und der Messelektrode 2, weist das Loch Lo auf |
| 8.2 | scheibenförmige Sperrfolie zwischen dem Oxidierungs-Bauteil 6 und der Referenzelektrode 5, 5, vom ringförmigen Spalt Sp umgeben |
| 9 | elastischer Stempel zwischen dem Oxidierungs-Bauteil 6 und der Abdeckung 11 11 in der Stützhülse 16 |
| 10 | Gehäuse, umschließt den Innenraum I, umfasst die messzellenseitige Öffnung Ö, ist mechanisch mit der Stützhülse 16 verbunden, nimmt u.a. die Messzelle und das Oxidierungs-Bauteil 6 auf |
| 11 | Abdeckung für das Gehäuse 10 |
| 12 | Elektrolyt zwischen den Elektroden 2 und 4 der Messzelle |
| 13 | gasdurchlässige Stützmembrane zwischen der Sperrschicht 1 und der Messelektrode 2 |
| 14 | Draht, der die Elektroden 2 und 4 elektrisch miteinander verbindet |
| 15 | Stromstärken-Sensor, misst die Stromstärke oder die elektrische Ladung des Stroms, der durch den Draht 14 fließt |
| 16 | Stützhülse um den elastischen Stempel 9, ist mechanisch mit der Wand W.2 des Gehäuses 10 verbunden, nimmt den Stempel 9 auf, lässt sich von der Abdeckung 11 verschließen, fungiert als Druckausgleichs-Auslass |
| 20 | Behälter zum Einfüllen des Elektrolyten E |
| 30 | Adapter, der lösbar auf die Öffnung Ö aufgesetzt werden kann |
| 31 | Schlauch, der mit dem Adapter 30 verbunden ist |
| 32 | Pumpe, der das Gas G durch den Schlauch 31 hindurch ansaugt |
| 100 | Sensor, umfasst die Elektroden 2, 4, 5, 6, das Gehäuse 10, die Sperrschichten 3, die Sperrfolie 8.1 und 8.2, den Stempel 9 und den Deckel 11 |
| E | flüssiges Elektrolyt, wird aus dem Behälter 20 von oben in den Sensor 100 eingefüllt |
| G | zu untersuchendes Gas, kann in die Richtungen R.1 und R.2 in den Innenraum I hinein fließen |
| I | Innenraum des Sensors 100, vom Gehäuse 10 umschlossen |
| Lo | Loch in der ringförmigen Sperrfolie 8.1 |
| Ö | Öffnung, durch die hindurch Gas G durch die Sperrschicht 1 hindurch in den Innenraum I hinein diffundiert, fungiert als messzellenseitiger Einlass |
| R.1 | Richtung, in welche zu untersuchendes Gas G durch die Öffnung Ö hindurch in den Innenraum I fließt |
| R.2 | Richtung, in welche zu untersuchendes Gas G durch die Stützhülse 16 hindurch in den Innenraum I fließt, ist der Richtung R.1 entgegengesetzt |
| Sp | Spalt zwischen der Sperrfolie 8.2 und dem Gehäuse 10 |
| W.1 | Wand des Gehäuses 10, in welche der messzellenseitige Einlass Ö eingelassen ist |
| W.2 | Wand des Gehäuses 10, mit welcher die Stützhülse 16 verbunden ist |

## Patentansprüche

1. Elektrochemischer Sensor (100) zur Detektion eines vorgegebenen Gas-Bestandteils in einem Gas (G) und / oder zur Messung der Konzentration des Gas-Bestandteils in dem Gas (G),
wobei der elektrochemische Sensor (100)
- ein Gehäuse (10), das einen Innenraum (I) umschließt,
- eine Messzelle (2, 4, 12, 15) im Innenraum (I),
- eine elektrische Messeinheit (15),
- einen messzellenseitigen Einlass (Ö) im Gehäuse (10) und
- ein Oxidierungs-Bauteil (6) im Innenraum (I)
umfasst,
wobei der messzellenseitige Einlass (Ö) eine Fluidverbindung zwischen dem Gehäuse (10) und einer Umgebung des elektrochemischen Sensors (100) herzustellen vermag,
wobei die Messzelle (2, 4, 12, 15)
- eine Messelektrode (2),
- eine Gegenelektrode (4) und
- eine elektrisch isolierende und ionisch leitende Schicht (12) zwischen den beiden Elektroden (2, 4)
umfasst,
wobei die Messzelle (2, 4, 12, 15) so ausgestaltet ist, dass an der Messzelle (2, 4, 12, 15) eine chemische Reaktion stattfindet, die
- von der Konzentration des Gas-Bestandteils im Innenraum (I) abhängt und
- eine messbare elektrische Größe beeinflusst, und
wobei die elektrische Messeinheit (15) dazu ausgestaltet ist, ein Maß für die beeinflusste elektrische Größe zu messen,
**dadurch gekennzeichnet, dass**
der elektrochemische Sensor (100) einen Druckausgleichs-Auslass (16) im Gehäuse (10) umfasst,
wobei der Druckausgleichs-Auslass (16) eine Fluidverbindung zwischen dem Gehäuse (10) und einer Umgebung des elektrochemischen Sensors (100) herzustellen vermag,
wobei die Messzelle (2, 4, 12, 15) zwischen dem messzellenseitigen Einlass (Ö) und dem Druckausgleichs-Auslass (16) angeordnet ist und
wobei das Oxidierungs-Bauteil (6)
- zwischen dem Druckausgleichs-Auslass (16) und der Messzelle (2, 4, 12, 15) und mit einem Abstand zur Messzelle (2, 4, 12, 15) angeordnet ist,
- mindestens eine Elektrode umfasst, bevorzugt zwei Elektroden (6.1, 6.2),
- von der Messzelle (2, 4, 12, 15) elektrisch isoliert ist,
- so angeordnet und dazu ausgestaltet ist, mindestens einen, bevorzugt jeden oxidierbaren Bestandteil in dem Gas (G) zu oxidieren, das durch den Druckausgleichs-Auslass (16) hindurch in den Innenraum (I) gelangt, und
- bevorzugt mit der Messzelle (2, 4, 12, 15) ionisch leitend verbunden ist.

2. Elektrochemischer Sensor (100) nach Anspruch 1,
**dadurch gekennzeichnet, dass**
die Messelektrode (2) ein elektrisches Potenzial aufweist und mindestens eine Elektrode (6.1, 6.2) des Oxidierungs-Bauteils (6)
- ein elektrisches Potenzial aufweist, das oberhalb des elektrischen Potenzials der Messelektrode (2) liegt.

3. Elektrochemischer Sensor (100) nach einem der vorhergehenden Ansprüche,
**dadurch gekennzeichnet, dass**
der elektrochemische Sensor (100) einen Volumenfluss-Reduzierer (1) zwischen dem messzellenseitigen Einlass (Ö) und der Messzelle (2, 4, 12, 15) umfasst,
wobei der Volumenfluss-Reduzierer (1) dazu ausgestaltet ist, den Volumenfluss von der Umgebung durch den messzellenseitigen Einlass (Ö) hindurch zur Messzelle (2, 4, 12, 15) - verglichen mit einem Zustand ohne Volumenfluss-Reduzierer (1) - um mindestens 95 %, bevorzugt um mindestens 99 %, besonders bevorzugt um mindestens 99,9 %, insbesondere um mindestens 99,99 %, zu reduzieren.

4. Elektrochemischer Sensor (100) nach einem der vorhergehenden Ansprüche,
**dadurch gekennzeichnet, dass**
der elektrochemische Sensor (100) eine Referenzelektrode (5) umfasst, welche ein konstantes elektrisches Potenzial aufweist,
wobei das Oxidierungs-Bauteil (6)
- zwischen der Referenzelektrode (5) und dem Druckausgleichs-Auslass (16) angeordnet ist,
bevorzugt mit einem Abstand zur Referenzelektrode (5), und
- mit der Referenzelektrode (5) ionisch leitend verbunden ist.

5. Elektrochemischer Sensor (100) nach einem der vorhergehenden Ansprüche,
**dadurch gekennzeichnet, dass**
der elektrochemische Sensor (100) so ausgestaltet ist, dass ein flüssiges Elektrolyt (E) vom Druckausgleichs-Auslass (16) am Oxidierungs-Bauteil (6) vorbei zur Messzelle (2, 4, 12, 15) fließen kann.

6. Elektrochemischer Sensor (100) nach einem der vorhergehenden Ansprüche,
**dadurch gekennzeichnet, dass**
zwischen dem Druckausgleichs-Auslass (16) und der Messzelle (2, 4, 12, 15) mindestens eine für Gas undurchlässige Sperrschicht (8.1, 8.2) angeordnet ist,
wobei in die oder jede Sperrschicht (8.1, 8.2) jeweils mindestens eine für Gas (G) durchlässige Durchgangsöffnung (Sp, Lo) eingelassen ist.

7. Elektrochemischer Sensor (100) nach einem der vorhergehenden Ansprüche,
**dadurch gekennzeichnet, dass**
der messzellenseitige Einlass (Ö) und der Druckausgleichs-Auslass (16) in zwei einander gegenüberliegenden Wänden (W.1, W.2) des Gehäuses (10) eingelassen sind und
die Messzelle (2, 4, 12, 15) und das Oxidierungs-Bauteil (6) zwischen diesen beiden einander gegenüberliegenden Wänden (W.1, W.2) angeordnet sind.

8. Anordnung umfassend
- einen elektrochemischen Sensor (100) nach einem der vorhergehenden Ansprüche,
- einen Adapter (30) und
- eine Fluidführungseinheit (31),
wobei die Fluidführungseinheit (31) fluiddicht mit dem Adapter (30) verbunden ist und
wobei der Adapter (30) sich dergestalt lösbar auf das Gehäuse (10) aufsetzen lässt,
dass bei aufgesetztem Adapter (30) der messzellenseitige Einlass (Ö) nur durch die Fluidführungseinheit (31) hindurch mit einer Umgebung des elektrochemischen Sensors (100) in einer Fluidverbindung steht.

9. Verfahren zur Detektion eines vorgegebenen Gas-Bestandteils in einem Gas (G) und / oder zur Messung der Konzentration des Gas-Bestandteils in dem Gas (G),
wobei das Verfahren unter Verwendung eines elektrochemischen Sensors (100) durchgeführt wird,
wobei der elektrochemische Sensor (100)
- ein Gehäuse (10), das einen Innenraum (I) umschließt,
- eine Messzelle (2, 4, 12, 15) im Innenraum (I),
- eine elektrische Messeinheit (15),
- einen messzellenseitigen Einlass (Ö) im Gehäuse (10) und
- ein Oxidierungs-Bauteil (6) im Innenraum (I)
umfasst,
wobei der messzellenseitige Einlass (Ö) wenigstens zeitweise eine Fluidverbindung zwischen dem Gehäuse (10) und einer Umgebung des elektrochemischen Sensors (100) herstellt,
wobei die Messzelle (2, 4, 12, 15)
- eine Messelektrode (2),
- eine Gegenelektrode (4) und
- eine elektrisch isolierende und ionisch leitende Schicht (12) zwischen den beiden Elektroden (2, 4)
umfasst und
wobei das Verfahren die Schritte umfasst, dass
- eine Probe des Gases (G) durch den messzellenseitigen Einlass (Ö) hindurch in den Innenraum (I) fließt und die Messzelle (2, 4, 12, 15) erreicht,
- an der Messzelle (2, 4, 12, 15) eine chemische Reaktion stattfindet, die von der Konzentration des Gas-Bestandteils im Innenraum (I) abhängt und eine messbare elektrische Größe beeinflusst und
- die elektrische Messeinheit (15) ein Maß für die beeinflusste elektrische Größe misst,
**dadurch gekennzeichnet, dass**
der elektrochemische Sensor (100) einen Druckausgleichs-Auslass (16) im Gehäuse (10) umfasst,
wobei der Druckausgleichs-Auslass (16) wenigstens zeitweise eine Fluidverbindung zwischen dem Gehäuse (10) und einer Umgebung des elektrochemischen Sensors (100) herstellt,
wobei die Messzelle (2, 4, 12, 15) zwischen dem messzellenseitigen Einlass (Ö) und dem Druckausgleichs-Auslass (16) angeordnet ist,
wobei das Oxidierungs-Bauteil (6)
- zwischen dem Druckausgleichs-Auslass (16) und der Messzelle (2, 4, 12, 15) und mit einem Abstand zur Messzelle (2, 4, 12, 15) angeordnet ist,
- mindestens eine Elektrode umfasst, bevorzugt zwei Elektroden (6.1, 6.2), und
- von der Messzelle (2, 4, 12, 15) elektrisch isoliert ist und
bevorzugt mit der Messzelle (2, 4, 12, 15) ionisch leitend verbunden ist und
wobei das Verfahren den Schritt umfasst, dass
das Oxidierungs-Bauteil (6) mindestens einen, bevorzugt jeden oxidierbaren Bestandteil in dem Gas (G) oxidiert, das durch den Druckausgleichs-Auslass (16) hindurch in den Innenraum (I) gelangt.

## Claims

1. Electrochemical sensor (100) for detecting a predetermined gas component in a gas (G) and/or for measuring the concentration of the gas component in the gas (G),
wherein the electrochemical sensor (100) comprises
- a housing (10) that encloses an interior space (I),
- a measuring cell (2, 4, 12, 15) in the interior space (I),
- an electrical measuring unit (15),
- a measuring-cell-side inlet (Ö) in the housing (10) and
- an oxidation component (6) in the interior space (I),
wherein the measuring-cell-side inlet (Ö) is capable of establishing a fluid connection between the housing (10) and an environment of the electrochemical sensor (100),
wherein the measuring cell (2, 4, 12, 15) comprises
- a measuring electrode (2),
- a counter electrode (4) and
- an electrically insulating and ionically conductive layer (12) between the two electrodes (2, 4),
wherein the measuring cell (2, 4, 12, 15) is designed in such a way that a chemical reaction takes place at the measuring cell (2, 4, 12, 15), which
- depends on the concentration of the gas component in the interior space (I) and
- influences a measurable electrical quantity, and
wherein the electrical measuring unit (15) is designed to measure a measure of the influenced electrical quantity,
**characterized in that**
the electrochemical sensor (100) comprises a pressure equalization outlet (16) in the housing (10),
wherein the pressure equalization outlet (16) is capable of establishing a fluid connection between the housing (10) and an environment of the electrochemical sensor (100),
wherein the measuring cell (2, 4, 12, 15) is arranged between the measuring-cell-side inlet (Ö) and the pressure equalization outlet (16) and
wherein the oxidation component (6)
- is arranged between the pressure equalization outlet (16) and the measuring cell (2, 4, 12, 15) and at a distance from the measuring cell (2, 4, 12, 15),
- comprises at least one electrode, preferably two electrodes (6.1, 6.2),
- is electrically insulated from the measuring cell (2, 4, 12, 15),
- is arranged and designed to oxidize at least one, preferably every oxidizable component in the gas (G) which passes through the pressure equalization outlet (16) into the interior space (I), and
- is preferably connected to the measuring cell (2, 4, 12, 15) in an ionically conductive manner.

2. Electrochemical sensor (100) according to claim 1,
**characterized in that**
the measuring electrode (2) has an electrical potential and
at least one electrode (6.1, 6.2) of the oxidation component (6) has an electrical potential which is above the electrical potential of the measuring electrode (2).

3. Electrochemical sensor (100) according to any of the preceding claims,
**characterized in that**
the electrochemical sensor (100) comprises a volume flow reducer (1) between the measuring-cell-side inlet (Ö) and the measuring cell (2, 4, 12, 15),
wherein the volume flow reducer (1) is designed to reduce the volume flow from the environment through the measuring-cell-side inlet (Ö) to the measuring cell (2, 4, 12, 15) - compared to a state without the volume flow reducer (1) - by at least 95%, preferably by at least 99%, particularly preferably by at least 99.9%, in particular by at least 99.99%.

4. Electrochemical sensor (100) according to any of the preceding claims,
**characterized in that**
the electrochemical sensor (100) comprises a reference electrode (5),
which has a constant electrical potential,
wherein the oxidation component (6)
- is arranged between the reference electrode (5) and the pressure equalization outlet (16), preferably at a distance from the reference electrode (5), and
- is connected to the reference electrode (5) in an ionically conductive manner.

5. Electrochemical sensor (100) according to any of the preceding claims,
**characterized in that**
the electrochemical sensor (100) is designed so that a liquid electrolyte (E) can flow from the pressure equalization outlet (16) past the oxidation component (6) to the measuring cell (2, 4, 12, 15).

6. Electrochemical sensor (100) according to any of the preceding claims,
**characterized in that**
at least one barrier layer (8.1, 8.2) which is impermeable to gas is arranged between the pressure equalization outlet (16) and the measuring cell (2, 4, 12, 15),
wherein at least one passage opening (Sp, Lo) which is permeable to gas (G) is formed in the or each barrier layer (8.1, 8.2).

7. Electrochemical sensor (100) according to any of the preceding claims,
**characterized in that**
the measuring-cell-side inlet (Ö) and the pressure equalization outlet (16) are formed in two mutually opposite walls (W.1, W.2) of the housing (10) and
the measuring cell (2, 4, 12, 15) and the oxidation component (6) are arranged between these two opposite walls (W.1, W.2).

8. Assembly comprising
- an electrochemical sensor (100) according to any of the preceding claims,
- an adapter (30) and
- a fluid guide unit (31),
wherein the fluid guide unit (31) is connected to the adapter (30) in a fluid-tight manner, and
wherein the adapter (30) can be detachably mounted on the housing (10) in such a way
that, when the adapter (30) is mounted, the measuring-cell-side inlet (Ö) is in fluid communication with an environment of the electrochemical sensor (100) only through the fluid guide unit (31).

9. Method for detecting a predetermined gas component in a gas (G) and/or for measuring the concentration of the gas component in the gas (G),
wherein the method is carried out using an electrochemical sensor (100),
wherein the electrochemical sensor (100) comprises
- a housing (10) that encloses an interior space (I),
- a measuring cell (2, 4, 12, 15) in the interior space (I),
- an electrical measuring unit (15),
- a measuring-cell-side inlet (Ö) in the housing (10) and
- an oxidation component (6) in the interior space (I),
wherein the measuring-cell-side inlet (Ö) at least temporarily establishes a fluid connection between the housing (10) and an environment of the electrochemical sensor (100),
wherein the measuring cell (2, 4, 12, 15) comprises
- a measuring electrode (2),
- a counter electrode (4) and
- an electrically insulating and ionically conductive layer (12) between the two electrodes (2, 4)
and
wherein the method comprises the steps whereby
- a sample of the gas (G) flows through the measuring-cell-side inlet (Ö) into the interior space (I) and reaches the measuring cell (2, 4, 12, 15),
- a chemical reaction takes place at the measuring cell (2, 4, 12, 15), which depends on the concentration of the gas component in the interior space (I) and influences a measurable electrical quantity and
- the electrical measuring unit (15) measures a measure of the influenced electrical quantity,
**characterized in that**
the electrochemical sensor (100) comprises a pressure equalization outlet (16) in the housing (10),
wherein the pressure equalization outlet (16) at least temporarily establishes a fluid connection between the housing (10) and an environment of the electrochemical sensor (100),
wherein the measuring cell (2, 4, 12, 15) is arranged between the measuring-cell-side inlet (Ö) and the pressure equalization outlet (16),
wherein the oxidation component (6)
- is arranged between the pressure equalization outlet (16) and the measuring cell (2, 4, 12, 15) and at a distance from the measuring cell (2, 4, 12, 15),
- comprises at least one electrode, preferably two electrodes (6.1, 6.2), and
- is electrically insulated from the measuring cell (2, 4, 12, 15) and
is preferably connected to the measuring cell (2, 4, 12, 15) in an ionically conductive manner and
wherein the method comprises the step whereby
the oxidation component (6) oxidizes at least one, preferably every oxidizable component in the gas (G) which passes through the pressure equalization outlet (16) into the interior space (I).

## Revendications

1. Capteur électrochimique (100) permettant la détection d'un constituant gazeux prédéterminé dans un gaz (G) et/ou la mesure de la concentration du constituant gazeux dans le gaz (G),
dans lequel le capteur électrochimique (100) comprend
- un boîtier (10) qui renferme un espace intérieur (I),
- une cellule de mesure (2, 4, 12, 15) dans l'espace intérieur (I),
- une unité de mesure électrique (15),
- une entrée côté cellule de mesure (Ö) dans le boîtier (10) et
- un composant d'oxydation (6) dans l'espace intérieur (I),
dans lequel l'entrée côté cellule de mesure (Ö) est capable d'établir une liaison fluidique entre le boîtier (10) et un environnement du capteur électrochimique (100),
dans lequel la cellule de mesure (2, 4, 12, 15) comprend
- une électrode de mesure (2),
- une contre-électrode (4) et
- une couche (12) électriquement isolante et ioniquement conductrice entre les deux électrodes (2, 4),
dans lequel la cellule de mesure (2, 4, 12, 15) est configurée de sorte qu'une réaction chimique se produit au niveau de la cellule de mesure (2, 4, 12, 15), laquelle réaction chimique
- dépend de la concentration du constituant gazeux dans l'espace intérieur (I) et
- influence une grandeur électrique pouvant être mesurée, et
dans lequel l'unité de mesure électrique (15) est configurée pour mesurer une mesure de la grandeur électrique influencée,
**caractérisé en ce que**
le capteur électrochimique (100) comprend une sortie de compensation de pressions (16) dans le boîtier (10),
dans lequel la sortie de compensation de pressions (16) est capable d'établir une liaison fluidique entre le boîtier (10) et un environnement du capteur électrochimique (100),
dans lequel la cellule de mesure (2, 4, 12, 15) est disposée entre l'entrée côté cellule de mesure (Ö) et la sortie de compensation de pressions (16) et
dans lequel le composant d'oxydation (6)
- est disposé entre la sortie de compensation de pressions (16) et la cellule de mesure (2, 4, 12, 15) et à une certaine distance de la cellule de mesure (2, 4, 12, 15),
- comprend au moins une électrode, de préférence deux électrodes (6.1, 6.2),
- est isolé électriquement de la cellule de mesure (2, 4, 12, 15),
- est disposé et conçu pour oxyder au moins un, de préférence chaque constituant oxydable dans le gaz (G) qui rejoint l'espace intérieur (I) en passant à travers la sortie de compensation de pressions (16), et
- de préférence, est relié de manière ioniquement conductrice à la cellule de mesure (2, 4, 12, 15).

2. Capteur électrochimique (100) selon la revendication 1,
**caractérisé en ce que**
l'électrode de mesure (2) présente un potentiel électrique et
au moins une électrode (6.1, 6.2) du composant d'oxydation (6) présente un potentiel électrique qui est supérieur au potentiel électrique de l'électrode de mesure (2).

3. Capteur électrochimique (100) selon l'une des revendications précédentes,
**caractérisé en ce que**
le capteur électrochimique (100) comprend un réducteur de débit volumique (1) entre l'entrée côté cellule de mesure (Ö) et la cellule de mesure (2, 4, 12, 15),
dans lequel le réducteur de débit volumique (1) est configuré pour réduire le débit volumique de l'environnement à travers l'entrée côté cellule de mesure (Ö) vers la cellule de mesure (2, 4, 12, 15), par rapport à un état sans réducteur de débit volumique (1), d'au moins 95 %, de préférence d'au moins 99 %, de manière particulièrement préférée d'au moins 99,9 %, en particulier d'au moins 99,99 %.

4. Capteur électrochimique (100) selon l'une des revendications précédentes,
**caractérisé en ce que**
le capteur électrochimique (100) comprend une électrode de référence (5),
laquelle présente un potentiel électrique constant,
dans lequel le composant d'oxydation (6)
- est disposé entre l'électrode de référence (5) et la sortie de compensation de pressions (16), de préférence à une certaine distance de l'électrode de référence (5), et
- est relié de manière ioniquement conductrice à l'électrode de référence (5).

5. Capteur électrochimique (100) selon l'une des revendications précédentes,
**caractérisé en ce que**
le capteur électrochimique (100) est configuré de telle sorte qu'un électrolyte liquide (E) peut s'écouler depuis la sortie de compensation de pressions (16) vers la cellule de mesure (2, 4, 12, 15) en passant par le composant d'oxydation (6).

6. Capteur électrochimique (100) selon l'une des revendications précédentes,
**caractérisé en ce que**
entre la sortie de compensation de pressions (16) et la cellule de mesure (2, 4, 12, 15) est disposée au moins une couche d'arrêt (8.1, 8.2) imperméable au gaz,
dans lequel respectivement au moins une ouverture de passage (Sp, Lo) perméable au gaz (G) est encastrée dans la ou chaque couche d'arrêt (8.1, 8.2).

7. Capteur électrochimique (100) selon l'une des revendications précédentes,
**caractérisé en ce que**
l'entrée côté cellule de mesure (Ö) et la sortie de compensation de pressions (16) sont encastrées dans deux parois (W.1, W.2) du boîtier (10) qui sont opposées l'une à l'autre et
la cellule de mesure (2, 4, 12, 15) et le composant d'oxydation (6) sont disposés entre lesdites deux parois (W.1, W.2) opposées l'une à l'autre.

8. Système comprenant
- un capteur électrochimique (100) selon l'une des revendications précédentes,
- un adaptateur (30) et
- une unité de guidage de fluide (31),
dans lequel l'unité de guidage de fluide (31) est connectée de manière étanche aux fluides à l'adaptateur (30), et
dans lequel l'adaptateur (30) peut ainsi être placé de manière amovible sur le boîtier (10),
en ce que, lorsque l'adaptateur (30) est en place, l'entrée côté cellule de mesure (Ö) est en liaison fluidique avec un environnement du capteur électrochimique (100) uniquement à travers l'unité de guidage de fluide (31).

9. Procédé permettant la détection d'un constituant gazeux prédéterminé dans un gaz (G) et/ou la mesure de la concentration du constituant gazeux dans le gaz (G),
dans lequel le procédé est mis en œuvre à l'aide d'un capteur électrochimique (100),
dans lequel le capteur électrochimique (100) comprend
- un boîtier (10) qui renferme un espace intérieur (I),
- une cellule de mesure (2, 4, 12, 15) dans l'espace intérieur (I),
- une unité de mesure électrique (15),
- une entrée côté cellule de mesure (Ö) dans le boîtier (10) et
- un composant d'oxydation (6) dans l'espace intérieur (I),
dans lequel l'entrée côté cellule de mesure (Ö) établit au moins temporairement une liaison fluidique entre le boîtier (10) et un environnement du capteur électrochimique (100),
dans lequel la cellule de mesure (2, 4, 12, 15) comprend
- une électrode de mesure (2),
- une contre-électrode (4) et
- une couche (12) électriquement isolante et ioniquement conductrice entre les deux électrodes (2, 4)
et
dans lequel le procédé comprend les étapes selon lesquelles
- un échantillon du gaz (G) s'écoule à travers l'entrée côté cellule de mesure (Ö) dans l'espace intérieur (I) et atteint la cellule de mesure (2, 4, 12, 15),
- une réaction chimique a lieu au niveau de la cellule de mesure (2, 4, 12, 15), laquelle réaction chimique dépend de la concentration du constituant gazeux dans l'espace intérieur (I) et influence une grandeur électrique pouvant être mesurée et
- l'unité de mesure électrique (15) mesure une mesure de la grandeur électrique influencée,
**caractérisé en ce que**
le capteur électrochimique (100) comprend une sortie de compensation de pressions (16) dans le boîtier (10),
dans lequel la sortie de compensation de pressions (16) établit au moins temporairement une liaison fluidique entre le boîtier (10) et un environnement du capteur électrochimique (100),
dans lequel la cellule de mesure (2, 4, 12, 15) est disposée entre l'entrée côté cellule de mesure (Ö) et la sortie de compensation de pressions (16),
dans lequel le composant d'oxydation (6)
- est disposé entre la sortie de compensation de pressions (16) et la cellule de mesure (2, 4, 12, 15) et à une certaine distance de la cellule de mesure (2, 4, 12, 15),
- comprend au moins une électrode, de préférence deux électrodes (6.1, 6.2), et
- est isolé électriquement de la cellule de mesure (2, 4, 12, 15) et
de préférence, est relié de manière ioniquement conductrice à la cellule de mesure (2, 4, 12, 15) et
dans lequel le procédé comprend l'étape selon laquelle
le composant d'oxydation (6) oxyde au moins un, de préférence chaque constituant oxydable dans le gaz (G) qui rejoint l'espace intérieur (I) en passant à travers la sortie de compensation de pressions (16).
